Europäisches Patentamt

European Patent Office

Office européen des brevets

(19)

(11) **EP 0 709 115 A1**

(12) **EUROPEAN PATENT APPLICATION**

(43) Date of publication:
01.05.1996 Bulletin 1996/18

(51) Int. Cl.$^6$: **A61N 2/00**

(21) Application number: **94203134.5**

(22) Date of filing: **27.10.1994**

(84) Designated Contracting States:
**DE DK GB IT**

(71) Applicants:
• **CONSIGLIO NAZIONALE DELLE RICERCHE**
**I-00185 Roma (IT)**
• **Ilmoniemi, Risto Juhani**
**SF-02150 Espoo (FI)**

(72) Inventors:
• **Grandori, Ferdinando**
**I-20126 Milano (IT)**
• **Ilmoniemi, Risto Juhani**
**SF-02150 Espoo (FI)**

(74) Representative: **Marchi, Massimo et al**
**c/o Marchi & Mittler s.r.l.**
**Viale Lombardia 20**
**I-20131 Milano (IT)**

(54) **Device for applying a programmable excitation electric field to a target**

(57)     A device for applying a programmable excitation electric field to a target (P) comprises a plurality of elements (9; 10) for producing a plurality of single electric fields which are placed at predetermined locations into said target (P); the elements (9; 10) are formed by coils (9) and/or couples of electrodes (10) operatively connected to electric energy feeding equipments (8) and to a control unit (11) capable of adjusting the electric energy feeding to each of said elements (9; 10) for controlling the intensity of any single electric field and giving rise to a resultant excitation electric field which is the weighed sum of the single electric fields.

FIG. 1

## Description

The present invention relates to a device for applying an excitation electric field to a target, particularly for stimulation of the central and peripheral nervous systems of a subject and for therapy, tissue stimulation, hyperthermia applications and the like.

It is known that biological tissues can be influenced by the application of electromagnetic fields, which consist of magnetic fields **B** and of induced electric fields **E**. For example some tissues such as the brain and the heart can be stimulated with the help of an electric excitation field. The electric field **E** gives rise to an electric current $J(r)=\sigma(r)E(r)$, where $\sigma(\mathbf{r})$ is the conductivity of the tissue at a point determined by a vector **r**. This current may stimulate or otherwise influence the tissue in several different ways:

1) The charge distribution $\rho(r)$ is changed in the time t according to the curl of the electric current $\delta\rho/\delta t=-\nabla J$. This may cause large changes in the electric field according to $\nabla E= \rho/\varepsilon_o$, where $\varepsilon_o$ is the dielectric constant in the vacuum. As a result, a nerve membrane may depolarize and cause or facilitate the initiation of an action potential.

2) Since the current in tissue is carried mainly by ions, their concentration distribution will change. This may improve the healing of fractured or otherwise unhealthy tissue.

3) The temperature of the tissue will increase because of ohmic heating; the heating power P per unit volume V is $\Delta P/\Delta V= \sigma E^2$.

4) The volume element $\Delta V$ at r is subjected to the force $\Delta F=[J(r)xB]\Delta V$.

These effects may be utilized in several known ways:

a) By directing the stimulating electric field to a desired portion of a nerve or the brain, the condition of the nervous system may be diagnosed. If the cortical representation area for fingers, for example, is stimulated, movement of finger muscles can be observed after a signal-transmission delay. An abnormal or missing reaction may act as a useful diagnostic signal of neural malfunction.

b) By disturbing selected portions of the cortex or nerves of a subject performing a task it is possible to investigate how the healthy nervous system functions. If, for example, the visual cortex is stimulated after letters of the alphabet have been shown to the subject and this person is unable to recognize the characters of the words, one can conclude, under certain assumptions, that the stimulated part of the cortex has something to do with the processing of visual stimuli at the time the stimulus was delivered. One can thereby follow the path of a signal from the periphery toward the higher areas of the brain.

c) In hyperthermic treatment, a tissue is heated to a temperature in excess of 42°C.

d) Stimulation of tissue may have a therapeutic, pain-relieving, or curing effect.

e) Healing of certain tissue damages may be speeded up by magnetic stimulation.

It is known to set up an electric field **E** in tissues of a body by means of feeding current through electrodes which are applied to the body or by means of generating in the body a changing magnetic field **B(r,t)** in a coil which is placed near the body and is fed by a time-dependent current, e.g., releasing a charge stored in a condensator. The electric field **E** induced into the body is described by the Maxwell's equation $\nabla xE= - \delta B/\delta t$.

The current density distribution at a point determined by a vector **r'** of the coil conductors, $\mathbf{J_c(r',t)}$ gives rise to a magnetic field according to the Ampere-Laplace law:

$$B(r,t)=(\mu_o/4\pi)\int J_c(r',t)xR/R^3 dV, \qquad (1)$$

where **B(r,t)** is the magnetic flux density (here called magnetic field) at point determined by the vector **r** at time t. $R=r-r'$ is a vector from source point to field point. The magnetic field is the curl of the magnetic potential vector **A** according to $B= \nabla xA$. A changing magnetic field **B** induces an electric field **E** according to the Maxwell's equation: $\nabla \times E=- \delta B/\delta t$, as said above. **E** can be computed directly from $\mathbf{J_c}$ as follows:

$$E=- \delta A/\delta t=-(\mu_o/4\pi)\delta/\delta t\int J_c(r',t)/RdV. \qquad (2)$$

If the coil has been made from a thin wire and it carries the current I(t), the formula for the induced electric field is simple:

$$E=-(\mu_o/4\pi)\delta I(t)/\delta t\int_c dl/R, \qquad (3)$$

where the integration is made along a path c, along which the differential lenght element is dl.

The computation of the induced electric field is complicated by the fact that the arising electric current itself produces a secondary magnetic field.

Therefore, equations 1-3 are not exact; however, for typical frequency components involved in the magnetic excitation (10 kHz and below), when the target is a biological tissue and thus not a very good conductor, the error is rather small. At high frequencies the time-dependent terms in the Maxwell's equations must be fully taken into account.

It is also known to generate a more focused field distribution than that produced by a simple coil by means of an eight shaped coil, which consist of two adjacent oppositely wound coils. In an area which is near to the joining of the two coils (in close prossimity of a plane in respect of which the two coils are mirror images of each other) the fields generated by the coils add to each other, whereas further away from this area they partially calcel out each other. In this way a narrower field maximum is formed than with a single coil.

One drawback of the known devices is represented by the fact that the site of a target where an excitation electric field is applied can be changed only moving the electrodes or the coil/coils. This requires time and the positioning is not very precise, because the movements are performed manually by an operator.

Another drawback consists in that the operator gets no direct indication about the precise site of the target where the excitation electric field is applied.

A further drawback connected to the excitation coil/coils is represented by the fact that the pulsating feeding electric current heats considerably the coil/coils; thus the maximum frequency of the current must be limited.

An object of the present invention is a device which is able to focus an excitation electric and magnetic field in a desired location of a target better and more conveniently than the known devices allow.

Another object of the present invention is a device which is able to continously adjust maximum site, intensity and direction of stimulation of a target without moving means for generating an excitation electric and magnetic field.

A further object of the present invention is a device which is able to visualize to an operator maximum site and distribution of an excitation field.

Said objects are attained, according to the invention, by means of a device for applying a programmable excitation electric field to a target comprising generating means for producing said excitation electric field operatively connected to electric energy feeding means, characterized in that said generating means comprise a plurality of elements for producing a plurality of single electric fields which are placed at predetermined locations into said target, said elements being operatively connected to said electric energy feeding means and to control means capable of adjusting the electric energy feeding to each of said elements for controlling the intensity of any single electric field and giving rise to a resultant excitation electric field which is the weighed sum of said single electric fields, said resultant excitation electric field having a predetermined intensity, being focused in at least a predetermined maximum site and being directed in a predetermined direction according to a preselected distribution of said resultant excitation electric field into said target.

According to an embodiment of the invention, said plurality of elements comprise excitation coils.

According to another embodiment of the invention, said plurality of elements comprise couples of excitation electrodes.

Preferably, said plurality of elements comprise excitation coils as well as couples of excitation electrodes.

According to a further embodiment of the invention, said control means are operatively connected to a programmed computer storing at least a program for performing preselected distributions of said resultant excitation electric field, said programmed computer being capable of outputting signals for adjusting the amount of electric energy supplied to each of said element for performing said preselected distribution according to input signals.

Advantageously, said programmed computer is operatively connected to target imaging means for displaying an image of said target and of said resultant excitation electric field, in order to address said resultant excitation electric field to at least a preselected location in said target.

The device according to the invention shows the advantage that the maximum and extremum of the induced electric field, the application site and the direction of the stimulation of the target can be continuously adjusted to a desired location without moving the coils and/or electrodes. The device comprises many excitation coils and/or electrodes which are placed at fixed locations and can be used selectively so that the resulting field can be directed and focused and to a desired point. In addition, the direction of the field can be oriented into a desired direction. These performances can be obtained by feeding into the different coils and electrodes such currents that the maximum and direction of the resultant field have the desired features. Residual or parasitic field components can be adjusted in certain limits at selected point where excitation should be avoided.

In the device according to the invention a rapidly changing magnetic field is produced in the target by means of a multiplicity of coils outside the target and the currents fed to the different coils is determined so that the distribution of the resulting induced field is as close as possible to the one specified by an operator.

Moreover, according to an embodiment of the device, electrodes are used in ohmic or capacitive contact with the target instead of or in addition to coils. Current fed through the electrodes give rise to changes in the electric field in the target. Thus it is possible to improve the focality or resolution of systems relying exclusively on magnetic stimulation. The current fed to the electrodes is selected so that the electric field in the target produced by them is in a desired phase relation (usually in phase) with respect to the phase of the field induced from magnetic stimulation. Since the magnetically induced field is proportional to the time derivative of the feeding current, the pulse shapes of electric and magnetic stimulation must be different.

Arranging a display, in which there are shown the excitation fields computed according to selected current distributions and amplitudes makes possible to an operator to see immediately where the excitation site is located and how it is distributed about the maximum.

The device according to the invention is suitable for a wide range of applications, because it makes possible choosing and adjusting electric and magnetic fields, producing the chosen field distribution and adjusting it continuously.

In particular, the device is advantageous for biological applications in which nerves, brain, heart or other excitable tissues of a subject are to be stimulated, e.g., for the purpose of research, diagnosis, therapy, or treatment. One application is formed by spatiotemporal temperature control systems, in which the temperature of a conducting medium is continuously and flexibly adjusted as a function of the location and time by applying into it an adjustable and focusable electric field for heating the medium either through electrodes or by means of electromagnetic induction. Hyperthermia is one medical application, in which a local raising of temperature can be used to destroy cancerous tissue. An operator can, for example, investigate point by point with test pulses how sensitive the extracranial skin and muscles are to the fringe fields. It is possible operate providing that the final excitation field may not cause any pain or muscle movements or eye movements or other disturbing or undesirable influences. The device gives the possibility to adjust the field shape in such a way that harmful or parasitic effects are on an acceptable level.

The device according to the invention makes possible also such stimulation sequences, in which successive stimuli are directed to different portions of the cerebral cortex without moving the coils. The first excitation pulse can be applied to one location, the second, after, e.g., 100 ms, can be applied to another location etc. This procedure allows the investigation of such dynamic cerebral phenomena, in which the transmission times and processing times are of this order of magnitude. It is also possible to deliver the stimuli at very short intervals (on the order of 1 ms) so that from the point of view of the brain they are virtually simultaneous. In this way, two or more sites can be excited essentially simultaneously even if they would be in the same or nearly the same place but oriented differently so that their simultaneous excitation would be impractical. One can also superpose stimuli to two or more populations of neural nuclei and deliver the multiple stimulation at once. In medical applications an image of the shape and amplitude of the excitation field can be superimposed on anatomical cross-sectional images of the target, such as magnetic resonance images. An operator can also deliver test stimuli and, on the basis of results, adjust the locus and shape of the stimulating field until delivering the final type of stimulus.

In an implementation of the device the coils are made small (diameter of the order of few centimeters) and their mutual distances small (few millimeters) so that they alone or together with the electrodes connected to the cortex can be used selectively and accurately to stimulate the cortex during brain surgery such as in the treatment of epilepsy. When the coils are placed close to the target, the required current amplitudes are considerably smaller than those required in usual extracranial stimulation. In addition, focusing is better in proportion to the distance from the coils to the cortex.

The possibility of focusing and directing the excitation field to a desired location can also be used in connection with magneto- and/or electroencephalography in such a way that the locus of field maximum is determined on the basis of measure signals from the brain. This allows that the field can be directed precisely to experimentally determined locations such as epiletic foci or parts of the motor cortex. Features and advantages of the invention will now be illustrated with reference to an embodiment represented as non-limiting example in the enclosed figures, wherein:

Fig. 1 is a block scheme of a device for applying a programmable excitation electric field to a target, according to the invention;

Fig. 2 shows three distributions of an electric field which is generated by the device of Fig. 1;

Fig. 3 shows a frame for supporting coils of the device of Fig. 1.

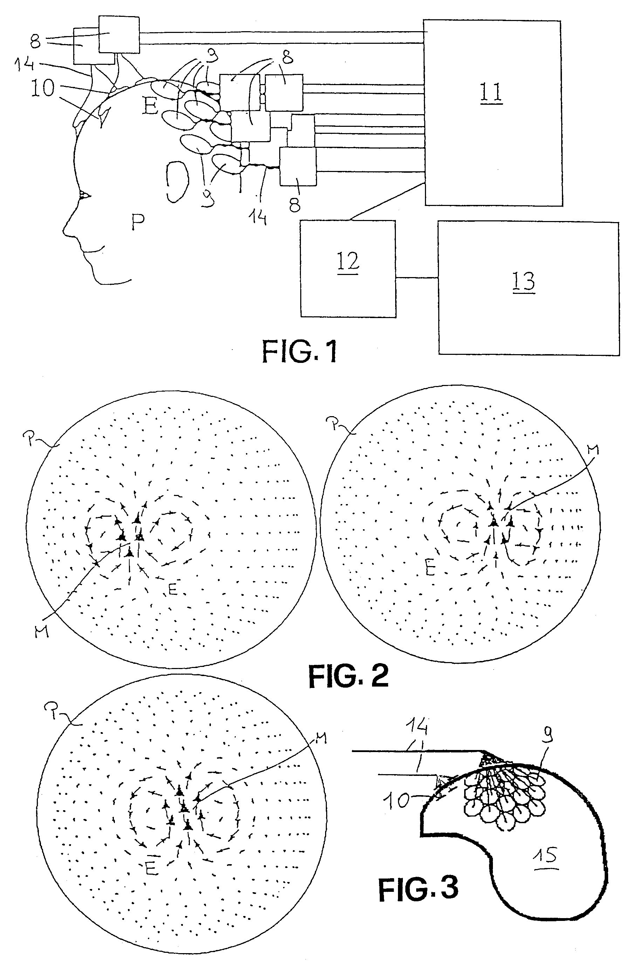

Fig. 1 shows schematically a device for applying a programmable excitation electric field to a target P which is embodied according to the invention.

The device comprises a plurality of elements 9 and 10 for producing a plurality of single electric fields which are placed at predetermined locations in said target P, which is the brain of a subject to be examined or treated. Said elements are formed by a plurality of excitation coils 9 as well as plurality of couples of excitation electrodes 10. The excitation coils 9 are spaced from the target P and are supported by a frame 15 formed by a helmet, as that shown in Fig. 3, which is worn by the subject. While, the electrodes 10 lie on the skin of the head of the subject. The coils 9 have diameter comprised in a range of 5 to 40 mm and are placed at a mutual distance comprised in a range of 5 to 50 mm; the couples of electrodes 10 have a mutual distance comprised in a range of 5 to 50 mm.

The excitation coils 9 and the excitation electrodes 10 are connected through conductors 14 to electric energy feeding equipments 8 which in turn are operatively connected to a control unit 11 and to a computing unit 12. The current amplitudes and waveforms of the various current feeding equipments 8 are controlled by the control unit 11. The control unit 11 comprises silicon controlled rectifiers (SCR) which are capable of adjusting the amount of electric energy supplied to each coil 9 and to each couple of electrodes 10 for regulating the intensity of any single electric field and giving rise to a resultant excitation electric field E which is the weighed sum of said single electric fields. The computing unit 12 comprises a programmed computer 12 which stores a program for performing preselected distributions of said resultant excitation electric field. Signals are inputted by an operator in the computer 12 which outputs signals for adjusting the electric energy supply in order to realize into the target P a preselected distribution of the resultant excitation electric field E. Thus the resultant excitation electric field E has a predetermined intensity, is focused in a predetermined maximum site or two or more predetermined maximum sites and is directed in a predetermined direction.

Particularly, the programmed computer 12 stores a program for changing the maximum site and direction of the resultant excitation electric field according to a predetermined stimulation sequence of the target P.

The programmed computer 12 is operatively connected to target imaging means 13 for displaying an image of the target P and of the resultant excitation electric field, in order to allows to an operator to address the resultant excitation electric field E to a preselected location or to two or more preselected locations in the target P.

The computer 12 is capable of calculating the current amplitude that is fed into each excitation coil and couple of electrodes. The starting parameter inputted by the operator are the desired site of maximal excitation $r_m$ and the desired direction of the field $e_m$ at that point. In a first step it is computed what kind of electric and magnetic fields would be produced if there would be a unit current dipole at $r$, oriented along $e_m$, i.e. the current $I = 1/\Delta L$ from $r$ to $r+\Delta Le_m$ so that the product of the current and the path length would be 1 (one). At point $r$ there would be a sink for current I and at point $r+ALe_m$ a source; the current would return from the source to the sink along the surrounding conducting medium. The electromagnetic field due to such a current dipole are computed automatically with known algorithms using the equations 1-3 written above when the conductivity distribution of the tissue is known. Another known algorithm is then used to form such a linear combination of current patterns resulting from each excitation coil and couple of electrode that this combined pattern would produce at the coils and electrodes the same magnetic and electric signals as the dipole. The resulting current distribution can be further trimmed interactively by the operator or automatically by the computer 12 until the desired properties of the stimulating field have been met or approximated as well as it is possible.

According to an implementation of the device, the operator is capable to adjust interactively the locus of the stimulation point and the parasitic field, noting how the peak field is changed when fringe fields are reduced. The computer 12 calculates the field distribution from knowledge of the currents fed into each stimulation coil and couple of electrodes and from a model of the distribution of conductivity in the target P.

By means of the device the focus of the resultant electric field is controlled by the computer 12, either completely automatically or interactively. For example, an operator can supply to the computer the location and direction of the desired current in the target, on the basis of which the computer will graphically display the best possible current distribution that can be focused to the given point in the given direction. If the operator believes that the fringe field is too strong at a place where excitation should be avoided, the operator can supply to the computer relative input signals and the program will modify the currents and present a new excitation field pattern. This adjustment can be continued as long as the operator wants. In some applications the graphic display of the current is superimposed on anatomical cross-sections of the target or on a three-dimensional representation of the target. From such anatomical three-dimensional images it will be easy for the operator to choose the desired excitation site and point out it directly from the image with a specific pointer devices such as cursor buttons.

A feature of the device according to the invention is represented by the fact that the electric field induced via the magnetic field is proportional to the rate of change of current in the exciting coils 9 and that the electric field produced with electrodes 10 is proportional to the current fed through them. In other words, the phenomenon is linear. If the current amplitude is increased, the shape of the field remains the same, only its amplitude is increased. The field produced by the set of coils or electrodes is the vector sum of the fields produced by the different coils and electrodes derivations separately.

Fig. 2 shows three different distributions of the resultant electric field E which are performed by means of the device of Fig. 1. The lenght of each arrow is proportional to the electric field at the center of the arrow, while the direction of the arrow shows the direction of the current. Said three distributions are obtained using three different mappings of the current feeding to the coils 9 and to the electrodes 10.

The excitation coils 9 and electrodes 10 have been mantained by the helmet 15 on a spherical surface having a 90 mm radius at 10 mm away from the surface of the conducting target P which is also assumed to be spherical and the amplitude distribution of the resultant electric field E has been computed on a spherical surface at 15 mm below the surface of the target P. The device according to the invention allows to move the maximum site M of the electric field E without moving the coils 9 and electrodes 10. It is possible also to vary the intensity and direction of the electric field likewise without moving coils 9 and electrodes 10. Moreover, the electric field E results even better focused than in the known devices.

According to an embodiment of the device, the coils 9 are made from superconducting material, in order to increase the excitation rate without problems of heating in the coils. For example, the excitation coils can be made from high-transition-temperature material. In this case, no significant power excitation occurs in the coils so that the pulse frequency is not limited by thermal heating. An additional advantage arising from superconductors consists in that the coils can be made thinner that normal-metal coils.

Advantageously, the excitation coils are equipped with cooling means, for example, with water cooling means. It is possible use higher pulse frequencies than that allowed without cooling.

The magnetic flux of the electromagnetic fields produced by the coils 9 is preferably guided by para-, dia-, and ferromagnetic means as well as with conducting means. In this way the flux can be focused as a narrower "beam" to the target than is possible with ordinary coils.

The device according to the invention allows to change from one operating session to another the number, relative locations, and orientations of the coils as well as the number and locations of electrodes.

## Claims

1. Device for applying a programmable excitation electric field to a target (P) comprising generating means (9; 10) for producing said excitation electric field operatively connected to electric energy feeding means (8), characterized in that said generating means (9; 10) comprise a plurality of elements (9; 10) for producing a plurality of single electric fields which are placed at predetermined locations into said target (P), said elements (9; 10) being operatively connected to said electric energy feeding means (8) and to control means (11) capable of adjusting the electric energy feeding to each of said elements (9; 10) for controlling the intensity of any single electric field and giving rise to a resultant excitation electric field which is the weighed sum of said single electric fields, said resultant excitation electric field having a predetermined intensity, being focused in at least a predetermined maximum site and being directed in a predetermined direction according to a preselected distribution of said resultant excitation electric field into said target (P).

2. Device according to claim 1, characterized in that said plurality of elements (9; 10) comprise a plurality of excitation coils (9) producing a plurality of single electromagnetic fields which induce said resultant excitation field into said target (P), said coils (9) having diameter comprised in a range of 5 to 40 mm and being placed at a mutual distance comprised in a range of 5 to 50 mm.

3. Device according to claim 1, characterized in that said plurality of elements (9; 10) comprise a plurality of couples of excitation electrodes (10) producing a plurality of single electric fields which give rise to said resultant excitation electric field into said target, said couples of electrodes (10) having a mutual distance comprised in a range of 5 to 50 mm.

4. Device according to claim 1, characterized in that said plurality of elements (9; 10) comprise excitation coils (9) as well as couples of excitation electrodes (10).

5. Device according to claim 2, characterized in that said excitation coils (9) are provided with cooling means.

6. Device according to claim 2, characterized in that said excitation coils (9) are made of a superconducting material.

7. Device according to claim 2, characterized in that said excitation coils (9) are provided with means for guiding the magnetic flux to said target.

8. Device according to claim 1, characterized in that said plurality of elements (9; 10) producing a plurality of single electric fields are supported by a frame (15).

9. Device according to claim 1, characterized in that said control means (11) are operatively connected to a programmed computer (12) storing at least a program for performing preselected distributions of said resultant excitation electric field, said programmed computer (12) being capable of outputting signals for adjusting the amount of electric energy supplied to each of said element (9; 10) for performing said preselected distribution according to input signals.

10. Device according to claim 9, characterized in that said programmed computer (12) is operatively connected to target imaging means (13) for displaying an image of said target (P) and of said resultant excitation electric field, in order to address said resultant excitation electric field to at least a preselected location in said target (P).

11. Device according to claim 9, characterized in that said programmed computer (12) stores a program for changing said maximum site and direction of said resultant excitation electric field according to a predetermined stimulation sequence of said target (P).

FIG. 1

FIG. 2

FIG. 3

European Patent Office

**EUROPEAN SEARCH REPORT**

Application Number

EP 94 20 3134

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | FR-A-2 676 930 (PEKARIC-NAD)<br>* the whole document *<br>--- | 1 | A61N2/00 |
| A | EP-A-0 181 053 (IRT)<br>* the whole document *<br>--- | 1 | |
| A | EP-A-0 217 011 (RODIER)<br>* the whole document *<br>----- | 1 | |

TECHNICAL FIELDS
SEARCHED      (Int.Cl.6)

A61N

The present search report has been drawn up for all claims

| Place of search | Date of completion of the search | Examiner |
|---|---|---|
| THE HAGUE | 26 April 1995 | Taccoen, J-F |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)